(19) Europäisches Patentamt
European Patent Office
Office européen des brevets

(11) Veröffentlichungsnummer: **0 477 631 B1**

(12) **EUROPÄISCHE PATENTSCHRIFT**

(45) Veröffentlichungstag der Patentschrift: **22.11.95**

(51) Int. Cl.[6]: **C07C 235/34**, A01N 37/18

(21) Anmeldenummer: **91115145.4**

(22) Anmeldetag: **07.09.91**

Teilanmeldung 95106286.8 eingereicht am 07/09/91.

Die Akte enthält technische Angaben, die nach dem Eingang der Anmeldung eingereicht wurden und die nicht in dieser Patentschrift enthalten sind.

# (54) Ortho-substituierte Phenylessigsäureamide.

(30) Priorität: **22.09.90 DE 4030038**

(43) Veröffentlichungstag der Anmeldung:
**01.04.92 Patentblatt 92/14**

(45) Bekanntmachung des Hinweises auf die Patenterteilung:
**22.11.95 Patentblatt 95/47**

(84) Benannte Vertragsstaaten:
**AT BE CH DE DK ES FR GB GR IT LI NL SE**

(56) Entgegenhaltungen:
**EP-A- 0 088 325** **EP-A- 0 310 954**
**EP-A- 0 354 571** **EP-A- 0 398 692**
**CH-A- 636 601** **DE-A- 2 808 317**

(73) Patentinhaber: **BASF Aktiengesellschaft**
**Carl-Bosch-Strasse 38**
**D-67063 Ludwigshafen (DE)**

(72) Erfinder: **Brand, Siegbert, Dr.**
**Eyersheimer Strasse 42**
**W-6701 Birkenheide (DE)**
Erfinder: **Ammermann, Eberhard, Dr.**
**Sachsenstrasse 3**
**W-6700 Ludwigshafen (DE)**
Erfinder: **Lorenz, Gisela, Dr.**
**Erlenweg 13**
**W-6730 Neustadt (DE)**
Erfinder: **Sauter, Hubert, Dr.**
**Neckarpromenade 20**
**W-6800 Mannheim 1 (DE)**
Erfinder: **Oberdorf, Klaus, Dr.**
**Gartenstrasse 4**
**W-6904 Eppelheim (DE)**
Erfinder: **Kardorff, Uwe, Dr.**
**D 3,4**
**W-6800 Mannheim 1 (DE)**
Erfinder: **Kuenast, Christoph, Dr.**
**Salierstrasse 2**
**W-6701 Otterstadt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue ortho-substituierte Phenylessigsäureamide der allgemeinen Formel I

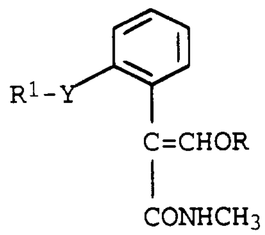

I,

in der die Variablen die folgende Bedeutung haben:

R eine $C_1$-$C_4$-Alkylgruppe,

$R^1$ Wasserstoff, eine $C_1$-$C_{18}$-Alkylgruppe,
eine $C_3$-$C_8$-Cycloalkylgruppe die noch einen bis drei Substituenten tragen kann, ausgewählt aus einer Gruppe von 3 Halogenatomen, 3 $C_1$-$C_4$-Alkylgruppen, einer partiell oder vollständig halogenierten $C_2$-$C_4$-Alkenylgruppe und einer Phenylgruppe, die noch ein bis zwei Halogenatome und/oder eine $C_1$-$C_4$-Alkylgruppe und/oder eine $C_1$-$C_4$-Alkoxygruppe tragen kann,
eine $C_2$-$C_{10}$-Alkenylgruppe,
eine $C_2$-$C_4$-Alkinylgruppe, die einen Phenylrest tragen kann,
eine $C_1$-$C_4$-Alkoxy-$C_1$-$C_4$-alkylgruppe, eine $C_1$-$C_4$-Alkoxycarbonylgruppe,
die Phenylgruppe, eine Phenyl-$C_1$-$C_4$-alkyl- oder Phenyl-$C_2$-$C_4$-alkenylgruppe oder eine Phenoxy-$C_1$-$C_4$-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: $C_1$-$C_4$-Alkyl, partiell oder vollständig halogeniertes $C_1$-$C_4$-Alkyl, $C_2$-$C_4$-Alkenyl, partiell oder voll- ständig halogeniertes $C_2$-$C_4$-Alkenyl und $C_1$-$C_4$-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder $C_1$-$C_4$-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff, Sauerstoff oder Schwefelatom annelliert sein kann und wobei der Heterocyclus zusätzlich noch ein Halogenatom, einen oder zwei $C_1$-$C_4$-Alkylreste oder einen Phenylrest tragen kann;

Y Sauerstoff, Schwefel,
eine Gruppe -SO-, $-SO_2-$, $-CH_2-O-SO_2-$, -N=N-, -O-CO- oder -CO-O-,
eine $C_1$-$C_4$-Alkylenkette, die partiell oder vollständig halogeniert sein kann, und die noch zusätzlich einen der folgenden Reste tragen kann: Cyano, Nitro, $C_1$-$C_4$-Alkyl,partiell oder vollständig halogeniertes $C_1$-$C_4$-Alkyl, $C_2$-$C_4$-Alkenyl, partiell oder vollständig halogeniertes $C_2$-$C_4$-Alkenyl, $C_1$-$C_4$-Alkoxy, Phenyl oder Phenoxy, wobei die letzten beiden Reste ihrerseits noch einen oder zwei der folgenden Substituenten tragen können: Cyano, Halogen oder $C_1$-$C_4$-Alkyl,
eine $C_2$-$C_4$-Alkenylen- oder $C_2$-$C_4$-Alkinylenkette, eine Oxy-($C_1$-$C_4$)-alkylen-, Thio-($C_1$-$C_4$)-alkylen- oder $C_1$-$C_4$-Alkylenoxykette oder eine Carbonyl-($C_1$-$C_4$)-alkylen- oder $C_1$-$C_4$-Alkylen-carbonylkette.

Außerdem betrifft die Erfindung Verfahren zur Herstellung dieser Verbindungen, ihre Verwendung als Fungizide und ihre Verwendung als Insektizide, Nematizide und Akarizide sowie fungizide Mittel und Mittel zur Bekämpfung von Schädlingen, welche diese Verbindungen als wirksame Substanzen enthalten.

Aus der EP-A 310 954 sind unter anderem fungizid wirksame ortho-substituierte Phenylessigsäureamide sowie deren Phenylacetonitril-Vorprodukte bekannt, wobei $R^1$ Wasserstoff, Phenyl oder 2,2-Dimethyl-3-(2',2'-dichlorvinyl)-cyclopropyl und Y Carboxymethylen bedeuten. Ähnliche Verbindungen sind aus EP 398 692 bekannt.

Der Erfindung lagen neue fungizid wirksame ortho-substituierte Phenylessigsäurederivate sowie neue insektizide, akarizide und nematizide Wirkstoffe als Aufgabe zugrunde.

Demgemäß wurden die eingangs definierten ortho-substituierten Phenylessigsäureamide der Formel I gefunden.

Im einzelnen haben die Substituenten in den erfindungsgemäßen Verbindungen I die folgende Bedeutung:

$R^1$

- Wasserstoff;
- eine verzweigte oder unverzweigte $C_1$-$C_{18}$-Alkylgruppe wie Methyl, Ethyl, n-Propyl, Isopropyl, 1,1-Dimethylprop-1-yl, 2,2-Dimethylprop-1-yl, n-Butyl, sec.-Butyl, Isobutyl, tert.-Butyl, 3-Methylbutyl, n-Pentyl, n-Hexyl, n-Heptyl, 2,6-Dimethylhept-1-yl, n-Octyl, n-Nonyl, n-Decyl, n-Pentadecyl, n-Heptadecyl und n-Octadecyl, bevorzugt eine $C_1$-$C_{10}$-Alkylgruppe;
- eine $C_3$-$C_8$-Cycloalkylgruppe wie Cyclopropyl, Cyclobutyl, Cyclopentyl, Cyclohexyl, Cycloheptyl und Cyclooctyl, die noch einen bis drei Substituenten tragen können, ausgewählt aus der einer Gruppe von 3 Halogenatomen wie Fluor, Chlor, Brom und Jod, insbesondere Fluor und Chlor, 3 $C_1$-$C_4$-Alkylgruppen wie Methyl, Ethyl, n-Propyl, Isopropyl, n-Butyl, sec.-Butyl und tert.-Butyl, einer partiell oder vollständig halogenierten $C_1$-$C_4$-Alkenylgruppe wie 2,2-Dichlorethenyl, und einer Phenylgruppe, die noch ein bis zwei Halogenatome wie vorstehend genannt, insbesondere Fluor und Chlor, und/oder eine $C_1$-$C_4$-Alkylgruppe wie Methyl, Ethyl, n-Propyl, Isopropyl, n-Butyl und tert.-Butyl und/oder eine $C_1$-$C_4$-Alkoxygruppe wie Methoxy, Ethoxy, n-Propoxy, Isopropoxy, n-Butoxy und tert.-Butoxy tragen kann; bevorzugt sind Cyclopropyl, 1-Methylcycloprop-1-yl, 2,2-Dichlorcycloprop-1-yl, (2',2'-Dichlorvinyl)-cyclopro-1-yl, 1-Phenylcycloprop-1-yl, 1-(p-Fluorphenyl)-cyclopro-1-yl, Cyclohexyl und 1-Methylcyclohex-1-yl;
- eine $C_2$-$C_{10}$-Alkenylgruppe wie Vinyl, Allyl, Prop-1-en-1-yl, Prop-2-en-2-yl, 2-Methylprop-1-en-1-yl, But-2-en-1-yl, But-2-en-2-yl, 3-Methylbut-2-en-1-yl, 1,3-Pentadien-1-yl, 2,6-Dimethylhept-5-en-1-yl und 2, 6-Dimethyl-1,5-heptadien-1-yl;
- eine $C_2$-$C_4$-Alkinylgruppe wie Ethinyl und Prop-2-in-1-yl, die noch einen Phenylrest tragen kann, z.B. 2-Phenylethinyl;
- eine $C_1$-$C_4$-Alkoxy-$C_1$-$C_4$-Alkylgruppe wie Methoxymethyl, Ethoxymethyl, n-Propoxymethyl, Isopropoxymethyl, n-Butoxymethyl, tert.-Butoxymethyl, 1-Methoxethyl, 2-Methoxyethyl, 1-Ethoxyethyl, 2-Ethoxyethyl und 2-n-Propoxyethyl);
- eine $C_1$-$C_4$-Alkoxycarbonylgruppe wie Methoxycarbonyl, Ethoxycarbonyl, n-Propoxycarbonyl, Isopropoxycarbonyl, n-Butoxycarbonyl und tert.Butoxycarbonyl, bevorzugt Methoxycarbonyl;
- die Phenylgruppe, eine Phenyl-$C_1$-$C_4$-alkylgruppe wie Benzyl, Phenethyl, 3-Phenyl-n-propyl und 4-Phenyl-n-butyl, eine Phenyl-$C_2$-$C_4$-alkenylgruppe wie Styryl und 2-Phenylprop-2-en-1-yl oder eine Phenoxy-$C_1$-$C_4$-alkylgruppe wie Phenoxymethyl, 2-Phenoxyethyl, 3-Phenoxypropyl und 4-Phenoxybutyl, wobei die genannten Gruppen am Phenylring jeweils noch insgesamt einen bis fünf Reste tragen können, davon insbesondere:
  - eine oder zwei Nitrogruppen,
  - eine oder zwei Cyanogruppen,
  - bis zu 5 Halogenatome wie vorstehend genannt, insbesondere Fluor und Chlor,
  - bis zu 3 $C_1$-$C_4$-Alkylgruppen wie vorstehend genannt,
  - bis zu 3 partiell oder vollständig halogenierte $C_1$-$C_4$-Alkylgruppen wie Fluormethyl, Chlormethyl, Trifluormethyl, Trichlormethyl, Dichlorfluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl,
  - bis zu 3 $C_2$-$C_4$-Alkenylgruppen wie Ethenyl, Prop-1-en-1-yl, Prop-2-en-1-yl, 1-Methylethen-1-yl, But-1-en-1-yl, But-2-en-1-yl, But-3-en-1-yl, 1-Methyl-prop-1-en-1-yl, 2-Methylprop-1-en-1-yl, 1-Methyl-pro-2-en-1-yl und 2-Methyl-prop-2-en-1-yl, insbesondere Ethenyl und Prop-2-en-1-yl,
  - bis zu 3 partiell oder vollständig halogenierte $C_2$-$C_4$-Alkenylgruppen wie 2-Fluorethenyl, 2-Chlorethenyl, Trifluorethenyl, Trichlorethenyl und 2-Chlor-prop-2-en-1-yl und
  - bis zu 3 $C_1$-$C_4$-Alkoxygruppen wie Methoxy, Ethoxy, n-Propoxy, Isopropoxy, n-Butoxy und tert.-Butoxy,
  - eine Phenyl-, Benzyl-, Phenoxy-, Benzyloxy- oder Phenylthiogruppe, die ihrerseits noch einen oder zwei der folgenden Substituenten tragen kann: Cyano, Halogen wie vorstehend genannt, insbesondere Fluor und Chlor oder $C_1$-$C_4$-Alkyl wie vorstehend genannt;

  bevorzugt sind Phenyl, 2-Nitrophenyl, 4-Nitrophenyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Jodphenyl, 2,3-Dichlorphenyl, 2,4-Dichlorphenyl, 2,5-Dichlorphenyl, 2,6-Dichlorphenyl, 2,3,4-Trichlorphenyl, 2,3,5-Trichlorphenyl, 2,3,6-Trichlorphenyl, 3,4,5-Trichlorphenyl, Pentafluorphenyl, Pentachlorphenyl, 2-Methylphenyl, 3-Methylphenyl, 4-Methylphenyl, 2-Ethylphenyl, 3-Ethylphenyl, 4-Ethylphenyl, 3-Isopropylphenyl, 4-Isopropylphenyl, 3-tert.-Butylphenyl, 4-tert.-Butylphenyl, 2,3-Dimethylphenyl, 2,4-Dimethylphenyl, 2,5-Dimethylphenyl, 3,4-Dimethylphenyl, 3,5-Dimethylphenyl, 4-tert.-Butyl-2-methylphenyl, 3,5-Diethylphenyl, 2,3,5-Trimethylphenyl, 2,4,6-Trimethylphenyl, 4-Cyclohexylphenyl, 3-Phenoxyphenyl, 4-Phenoxyphenyl, 4-Phenylthiophenyl, 3-Benzyloxyphenyl, 4-Benzyloxyphenyl, 2-Trifluormethylphenyl, 3-Trifluormethylphenyl, 4-Trifluormethylphenyl, 2-Chlormethylphenyl, 3-Chlormethylphenyl, 4-Chlormethylphenyl, Benzyl, 4-Chlorbenzyl, Phenethyl, 4-Chlorphenethyl, Styryl, 4-Chlorstyryl, Phenoxy, 2-Chlorphenoxy, 3-Chlorphenoxy, 4-Chlorphenoxy, 2-Methylphenoxy, 3-Methylphenoxy, 4-Methylphenoxy, 2-Trifluormethylphenoxy, 3-Trifluormethylphenoxy, 4-Trifluormethylphenoxy, Phenoxymethyl und 2-Phenoxyethyl;

- einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anelliert sein kann, beispielsweise Pyrrol-2-yl, Pyrrol-3-yl, Furan-2-yl, Furan-3-yl, Thien-2-yl, Thien-3-yl, Pyrazol-3-yl, Pyrazol-4-yl, Pyrazol-5-yl, Oxazol-2-yl, Oxazol-4-yl, Oxazol-5-yl, Benzoxazol-2-yl, Thiazol-2-yl, Thiazol-4-yl, Thiazol-5-yl, Benzothiazol-2-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-3-yl, 1,3,4-Triazol-2-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl und 1,2,4-Triazin-3-yl,

  wobei die Heterocyclen noch ein Halogenatom wie vorstehend genannt, insbesondere Fluor und Chlor, eine oder zwei $C_1$-$C_4$-Alkylgruppen wie vorstehend genannt, insbesondere Methyl oder einen Phenylrest tragen können, beispielsweise 5-Chlorbenzothiazol-2-yl, 6-Chlorpyridin-2-yl, 6-Methylpyridin-2-yl, 6-Ethylpyridin-2-yl, 6-n-Propylpyridin-2-yl, 6-Isopropylpyridin-2-yl, 6-n-Butylpyridin-2-yl, 6-sec.-Butylpyridin-2-yl und 6-tert.-Butylpyridin-2-yl, 6-Phenylpyridin-2-yl und 4,8-Dimethylchinolin-2-yl;

besonders bevorzugt werden Halogenphenyl, $C_1$-$C_4$-Alkylphenyl, Di-($C_1$-$C_4$)- alkylphenyl und Benzothiazol-2-yl;

y

- Sauerstoff oder Schwefel;
- eine Gruppe -SO-, $-SO_2-$, $-CH_2-O-SO_2-$, -N=N-, -O-CO- oder -CO-O-, bevorzugt -O-CO- und -CO-O-;
- eine $C_1$-$C_4$-Alkylenkette, die partiell oder vollständig halogeniert sein kann, insbesondere fluoriert oder chloriert, und die noch zusätzlich einen der folgenden Reste tragen kann: Cyano, Nitro, $C_1$-$C_4$-Alkyl wie vorstehend genannt, partiell oder vollständig halogeniertes $C_1$-$C_4$-Alkyl wie vorstehend genannt, $C_2$-$C_4$-Alkenyl wie vorstehend genannt, partiell oder vollständig halogeniertes $C_2$-$C_4$-Alkenyl wie vorstehend genannt, $C_1$-$C_4$-Alkoxy wie vorstehend genannt, Phenyl oder Phenoxy, wobei die letzten beiden Reste ihrerseits noch einen oder zwei der folgenden Substituenten tragen können: Cyano, Halogen wie vorstehend genannt, insbesondere Fluor und Chlor oder $C_1$-$C_4$-Alkyl wie vorstehend genannt, insbesondere Methyl; bevorzugt ist die Methylen- oder Ethylenkette;
- eine $C_2$-$C_4$-Alkenylenkette wie Ethenylen, Prop-2-enylen und But-2-enylen, bevorzugt Ethenylen;
- eine $C_2$-$C_4$-Alkinylenkette wie Ethinylen, Prop-2-inylen und But-2-inylen, bevorzugt Ethinylen;
- eine Oxy-($C_1$-$C_4$)-alkylenkette wie Oxymethylen, Oxyethylen, Oxy-n-propylen und Oxy-n-butylen, bevorzugt Oxymethylen;
- eine Thio-($C_1$-$C_4$)-alkylenkette wie Thiomethylen, Thioethylen, Thio-n-propylen und Thio-n-butylen, bevorzugt Thiomethylen;
- eine $C_1$-$C_4$-Alkylenoxykette wie Methylenoxy, Ethylenoxy, n-Propylenoxy und n-Butylenoxy, bevorzugt Methylenoxy;

- eine Carbonyl-($C_1$-$C_4$)-alkylenkette wie Carbonylmethylen, Carbonylethylen, Carbonyl-n-propylen und Carbonyl-n-butylen, bevorzugt Carbonylmethylen;
- eine $C_1$-$C_4$-Alkylencarbonylkette wie Methylencarbonyl, Ethylencarbonyl, n-Propylencarbonyl und n-Butylencarbonyl, bevorzugt Methylencarbonyl;

R

- eine $C_1$-$C_4$-Alkylgruppe wie Methyl, Ethyl, n-Propyl, Isopropyl, n-Butyl, sec.-Butyl und tert.-Butyl, bevorzugt Methyl;

Besonders geeignete ortho-substituierte Phenylessigsäureamide I sind Tabelle 1 zu entnehmen, wobei Verbindungen mit R = Methyl besonders bevorzugt sind.

Die Verbindungen I können bei der Herstellung als E/Z-Isomerengemische anfallen, wobei sich die beiden Isomeren durch die cis- oder trans-Stellung der Alkoxygruppe zum Säureamidteil unterscheiden. Die Isomeren können gewünschtenfalls nach den hierfür üblichen Methoden, z.B. durch Kristallisation oder Chromatographie, getrennt werden. Verbindungen mit E-Konfiguration (trans-Stellung der Alkoxygruppe der Alkoxymethylengruppe zum Säureamidteil) sind besonders bevorzugt.

Die ortho-substituierten Phenylessigsäureamide I sind auf verschiedene Weise erhältlich, und zwar vorzugsweise nach einer der folgenden Methoden:

a) Umsetzung von Phenylessigsäurederivaten II mit Aminen III

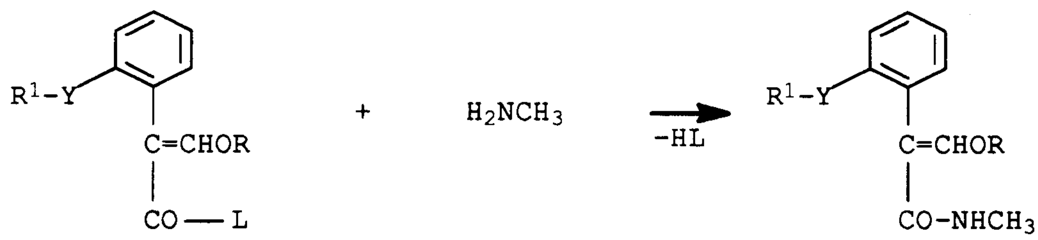


II III I

L bedeutet Halogen, insbesondere Chlor und Brom, oder $C_1$-$C_4$-Alkoxy, insbesondere Methoxy.

Die Umsetzung erfolgt normalerweise nach an sich bekannten Methoden (z.B. Organikum, 16. Auflage 1985, Seiten 4O9 bis 412) in einem inerten Lösungs- oder Verdünnungsmittel, vorteilhaft in Anwesenheit einer Base.

Als Lösungs- oder Verdünnungsmittel kommen insbesondere chlorierte Kohlenwasserstoffe wie Dichlormethan, Ether wie Dioxan sowie Alkohole wie Methanol und Ethanol in Betracht.

Als Basen eignen sich beispielsweise Alkalimetallhydroxide wie Natrium- und Kaliumhydroxid, Alkalimetallcarbonate wie Natrium- und Kaliumcarbonat, Alkalimetallalkoholate wie Natriummethylat und Natriumethylat, insbesondere tertiäre Amine wie Triethylamin und heteroaromatische Amine wie Pyridin und 4-Dimethylaminopyridin. Es kann aber auch das Amin III selbst als Base verwendet werden, und zwar für eine vollständige Umsetzung in mindestens der stöchiometrischen Menge, bezogen auf die Menge an II.

Zweckmäßig setzt man alle Ausgangsverbindungen in etwa stöchiometrischem Verhältnis ein, jedoch kann in manchen Fällen auch ein Überschuß der einen oder anderen Komponente, etwa bis zu 1O mol-%, empfehlenswert sein.

Verwendet man das Amin III als Base, so liegt es in einem größeren Überschuß vor.

Im allgemeinen liegt die Reaktionstemperatur zwischen 0 und 120 °C, insbesondere bei der Siedetemperatur des jeweiligen Lösungsmittels.

Bedeutet L Halogen, so ist die Durchführung der Reaktion auch in einem 2-Phasensystem unter Phasentransfer-Katalyse möglich. Dazu kann vorteilhaft eine Mischung aus einem chlorierten Kohlenwasserstoff wie Methylenchlorid, wäßriger Lauge, z.B. Natronlauge, und einem Phasentransferkatalysator wie Tetra-n-butylammoniumhydroxid verwendet werden. In diesem Fall arbeitet man z.B. bei Temperaturen zwischen 10 °C und der Siedetemperatur einer der Komponenten des Lösungsmittelgemisches.

Normalerweise arbeitet man bei Atmosphärendruck. Geringerer oder höherer Druck ist möglich, bringt aber im allgemeinen keine Vorteile.

Phenylessigsäurederivate II, wobei L Halogen bedeutet, sind bekannt oder können nach bekannten Verfahren dargestellt werden (z.B. Organikum, 16. Auflage 1985, Seiten 415, 622 und 423).

Die Phenylessigsäurederivate II, wobei L $C_1$-$C_4$-Alkoxy bedeutet, sind aus den Patentanmeldungen EP-A 178 826 und EP-A 226 917 bekannt oder können nach analogen Verfahren hergestellt werden.

Beispielsweise erhält man die Phenylessigsäurederivate II mit Y = Oxymethylen oder Thiomethylen durch nucleophile Substitution an Benzylhalogeniden VI

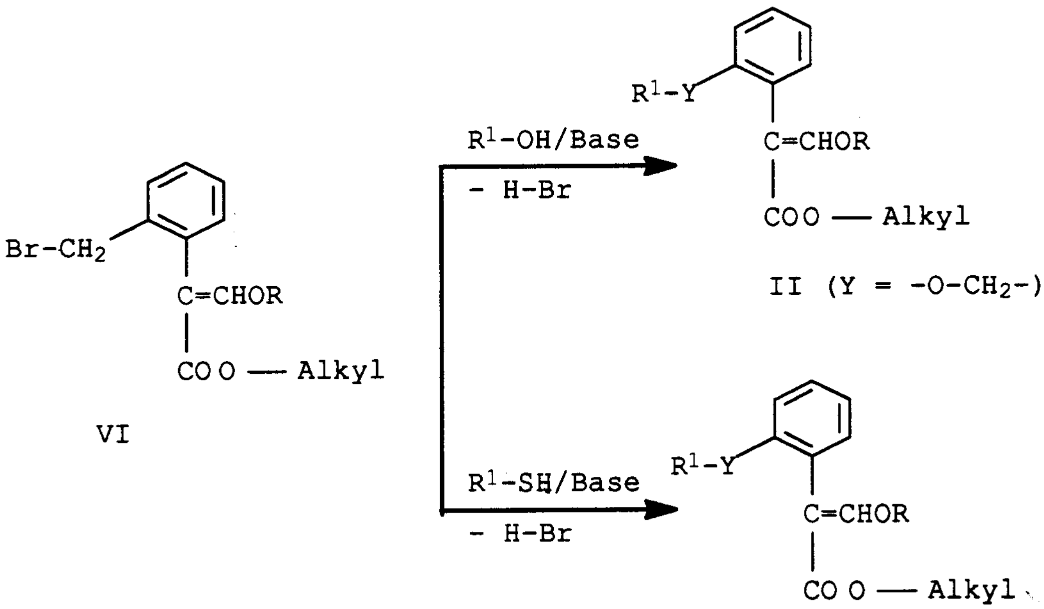


VI

II (Y = $-O-CH_2-$)

II (Y = $-S-CH_2-$))

b) Hydrolyse von Phenylacetonitrilen IV

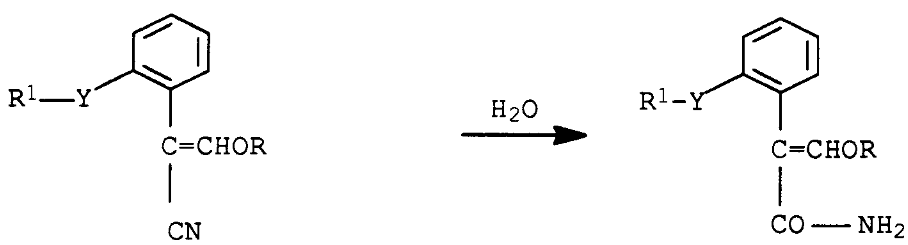


IV

V

Die Hydrolyse der Phenylacetonitrile IV erfolgt normalerweise säure- oder basenkatalysiert nach an sich bekannten Methoden (vgl. z.B. Beckwith in: Zabicky "The chemistry of Amides" Seiten 119 bis 125 (1970) und Synthesis, 243 (1980)] in einem inerten Lösungs- oder Verdünnungsmittel.

Als Lösungsmittel eignen sich insbesondere Alkohole wie tert.-Butanol und Ethylenglykol.

Als Säuren kommen bevorzugt konzentrierte Mineralsäuren wie Salzsäure, Schwefelsäure und Phosphorsäure in Betracht, als Basen bevorzugt Alkalimetallhydroxide wie Natrium- und Kaliumhydroxid.

Normalerweise liegt die Reaktionstemperatur zwischen 0 und 200 °C, insbesondere zwischen 20 °C und der Siedetemperatur des Lösungsmittels.

Bezüglich der Mengenverhältnisse und des Druckes gelten die Angaben für Methode (a).

Phenylacetonitrile der Formel IV sind beispielsweise aus der EP-A 310 954 bekannt oder können nach den dort beschriebenen Methoden hergestellt werden.

Die ortho-substituierten Phenylessigsäureamide V können nach an sich bekannten Verfahren [z.B. Challis in: Zabicky "The Chemistry of Amides", Seiten 731 bis 857 (1970)] am Stickstoff der Amidgruppe alkyliert werden:

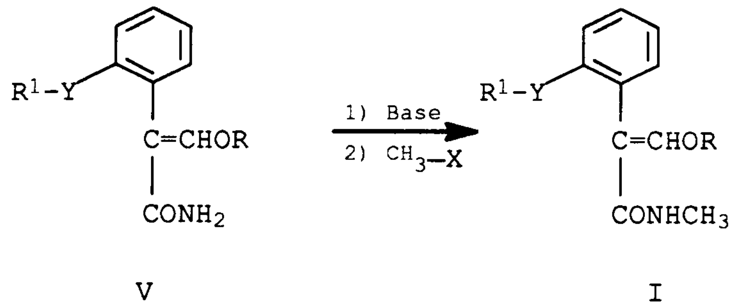


X bedeutet Halogen, insbesondere Brom und Jod.

Normalerweise überführt man hierzu die Phenylessigsäureamide V in einem inerten Lösungs- oder Verdünnungsmittel mittels Base in die Anionen und setzt diese mit einem Methylhalogenid, bevorzugt Methyljodid, um.

Als Lösungs- oder Verdünnungsmittel kommen bevorzugt Ether wie Tetrahydrofuran und Dioxan in Betracht.

Als Basen eignen sich insbesondere Alkalimetallhydroxide wie Natrium- und Kaliumhydroxid sowie Alkalimetallhydride wie Natrium- und Kaliumhydrid.

Im allgemeinen liegt die Reaktionstemperatur zwischen 0 und 100 °C, insbesondere bei der Siedetemperatur des jeweiligen Lösungsmittels.

Bezüglich der Mengenverhältnisse und des Druckes gelten die Angaben für Methode (a).

Die ortho-substituierten Phenylessigsäureamide I eignen sich als Fungizide und zur Bekämpfung von Schädlingen wie Insekten, Nematoden und Akaridien.

Die ortho-substituierten Phenylessigsäureamide I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmoparaviticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Die ortho-substituierten Phenylessigsäureamide I eignen sich des weiteren zur Bekämpfung von Schädlingen aus den Klassen der Insekten, Spinnentiere und Nematoden. Sie können im Pflanzenschutz

sowie auf dem Hygiene-, Vorratsschutz- und Veterinärsektor als Schädlingsbekämpfungsmittel eingesetzt werden.

Zu den schädlichen Insekten gehören:

- aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliaana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostriania nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni und Zeiraphera canadensis
- aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophagaundata, Bruchus rufimaanus, Bruchus pisorum, Bruchus leantis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicorniS, Diabrotica 12-punctata, Diabrotica virgifera, Epilachna varivestis, Epitrix hirtipennis, Eutianobothrus brasiliensis, Hylobius abietis, Hypera bruananeipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria;
- aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipieans, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Musciana stabulaans, Oestrus ovis, Oscianella frit, Pegomya hysocyami, Phorbia antiqua, Phorbia brassicae, Phorbia coarctata, Rhagoletis cerasi, Rhagoletis pomonella, Tabanus bovinus, Tipula oleracea und Tipulapaludosa;
- aus der Ordnung der Thripse (Thysanoptera) beispielsweise Frankliniella fusca, Frankliniella occideantalis, Frankliniella tritici, Scirtothrips citri, Thrips oryzae, Thrips palmi und Thrips tabaci;
- aus der Ordnung der Hautflügler (Hymenoptera) beispielsweise Athalia rosae, Atta cephalotes, Atta sexdeans, Atta texaana, Hoplocampa minuta, Hoplocampa testudinea, Monomorium pharaonis, Soleanopsis geminata und Soleanopsis invicta;
- aus der Ordnung der Wanzen (Heteroptera) beispielsweise Acrosternum hilare, Blissus leucopterus, Cyrtopeltis notatus, Dysdercus cingulatus, Dysdercus iantermedius, Eurygaster integriceps, Euchistus impictiventris, Leptoglossus phyllopus, Lygus lineolaris, Lygus pratensis, Nezara viridula, Piesma quadrata, Solubea insularis und Thyanta perditor;
- aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodoan humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum und Viteus vitifolii;
- aus der Ordnung der Termiten (Isoptera) beispielsweise Calotermes flavicollis, Leucotermes flavipes, Reticulitermes lucifugus und Termes natalensis

- aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum-Melanoplus mexicanus, Melanoplus sanguinipes, Melanop.lus spretus, Nomadacris septemfasciata, Periplaneta americana, Schistocerca americana, Schistocerca peregrina, Stauronotus maroccanus und Tachycines asynamorus;
- aus der Klasse der Arachanoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoani, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendlculatus, Rhipicephalus evertsi, Saccoptes scabiei, Tetranychus cinnabarinus, Tetranychus kanzawai, Tetranychus pacificus, Tetranychus telarius und Tetranychus urticae;
- aus der Klasse der Nematoden beispielsweise Wurzelgallennematoden, z.B. Meloidogyne hapla, Meloidogyne incoganita, Meloidogyne javanica, Zystean bildende Nematoden, z.B. Globodera rostochiensis, Heterodera avenae, Heterodera glycianae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Wirkstoffe können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßig Verteilung des ortho-substituierten Phenylessigsäureamids gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, öldispersionen durch Zusatz von Wasser bereitet werden. Zur Herstellung von Emulsionen, Pasten oder öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensatioansprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Wirkstoffkonzentrationen in den anwendungsfertigen Zubereitungen können in größeren Bereichen variiert werden.

Ganz allgemein enthalten die Mittel zwischen 0,0001 und 95, vorzugsweise zwischen 0,01 und 90 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Aufwandmengen in fungiziden Mitteln liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Insekten beträgt unter Freilandbedingungen 0,02 bis 10, vorzugsweise 0,1 bis 2,0 kg/ha.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln. Diese Mittel können zu den erfindungsgemäßen Mitteln im Gewichtsverhältnis 1 : 10 bis 10 : 1, gegebenenfalls auch erst unmittelbar vor der Anwendung (Tankmix), zugesetzt werden. Beim Vermischen mit Fungiziden oder insektiziden erhält man dabei in vielen Fällen eine Vergrößerung des Wirkungsspektrums.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Tabelle

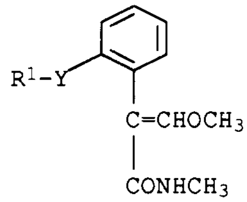

I

| Nr. | Y | $R^1$ |
|---|---|---|
| 1 | $CH_2$ | H |
| 2 | CHCl | H |
| 3 | CHBr | H |
| 4 | CHJ | H |
| 5 | $CH_2$ | OH |
| 6 | $CH_2$-O-$SO_2$ | $CH_3$ |
| 7 | $CH_2$-O-$SO_2$ | $C_6H_4$-$CH_3$ |
| 8 | $CH_2$-CH2 | $C_6H_5$ |
| 9 | $CH_2$-CH2 | 2-F-$C_6H_4$ |
| 10 | $CH_2$-CH2 | 3-F-$C_6H_4$ |
| 11 | $CH_2$-CH2 | 4-F-$C_6H_4$ |
| 12 | $CH_2$-CH2 | 2-Cl-$C_6H_4$ |
| 13 | $CH_2$-$CH_2$ | 3-Cl-$C_6H_4$ |
| 14 | $CH_2$-CH2 | 4-Cl-$C_6H_4$ |
| 15 | $CH_2$-CH2 | 2-Br-$C_6H_4$ |
| 16 | $CH_2$-$CH_2$ | 3-Br-C6$H_4$ |
| 17 | $CH_2$-$CH_2$ | 4-Br-$C_6H_4$ |
| 18 | $CH_2$-$CH_2$ | 2-J-$C_6H_4$ |
| 19 | $CH_2$-$CH_2$ | 2-$CH_3$-$C_6H_4$ |
| 20 | $CH_2$-$CH_2$ | 3-$CH_3$-$C_6H_4$ |
| 21 | $CH_2$-$CH_2$ | 4-$CH_3$-$C_6H_4$ |
| 22 | $CH_2$-$CH_2$ | 2-$OCH_3$-$C_6H_4$ |
| 23 | $CH_2$-$CH_2$ | 3-$OCH_3$-$C_6H_4$ |
| 24 | $CH_2$-$CH_2$ | 4-$OCH_3$-$C_6H_4$ |
| 25 | $CH_2$-$CH_2$ | 2-$CF_3$-$C_6H_4$ |
| 26 | $CH_2$-$CH_2$ | 3-$CF_3$-$C_6H_4$ |
| 27 | $CH_2$-$CH_2$ | 4-$CF_3$-$C_6H_4$ |
| 28 | $CH_2$-$CH_2$ | 4-i-$C_3H_7$-$C_6H_4$ |
| 29 | $CH_2$-CH2 | 4-t-$C_4H_9$-$C_6H_4$ |
| 30 | $CH_2$-$CH_2$ | 4-$C_6H_5$-$C_6H_4$ |

| Nr. | Y | $R^1$ |
|---|---|---|
| 31 | $CH_2-CH_2$ | 2,4-$Cl_2$-$C_6H_3$ |
| 32 | $CH_2-CH_2$ | 2,4-$(CH_3)_4$-$C_6H_3$ |
| 33 | $CH_2-CH_2$ | 2,4,6-$(CH_3)_3$-$C_6H_2$ |
| 34 | $CH_2-CH_2$ | 2,4,6-$Cl_3$-$C_6H_2$ |
| 35 | $CH_2-CH_2$ | Pyridin-2-yl |
| 36 | $CH_2-CH_2$ | Pyridin-3-yl |
| 37 | $CH_2-CH_2$ | Furan-2-yl |
| 38 | $CH_2-CH_2$ | 6-$CH_3$-Pyridin-2-yl |
| 39 | $CH_2-CH_2$ | 6-Cl-Pyridin-2-yl |
| 40 | $CH_2-CH_2$ | Benzothiazol-2-yl |
| 41 | CH=CH | $C_6H5$ |
| 42 | CH=CH | 2-F-$C_6H_4$ |
| 43 | CH=CH | 3-F-$C_6H_4$ |
| 44 | CH=CH | 4-F-$C_6H_4$ |
| 45 | CH=CH | 2-Cl-$C_6H_4$ |
| 46 | CH=CH | 3-Cl-$C_6H4$ |
| 47 | CH=CH | 4-Cl-$C_6H_4$ |
| 48 | CH=CH | 2-Br-$C_6H_4$ |
| 49 | CH=CH | 3-Br-$C_6H_4$ |
| 50 | CH=CH | 4-Br-C6H4 |
| 51 | CH=CH | 2-J-$C_6H_4$ |
| 52 | CH=CH | 2-$CH_3$-$C6H_4$ |
| 53 | CH=CH | 3-$CH_3$-$C_6H_4$ |
| 54 | CH=CH | 4-$CH_3$-$C6H_4$ |
| 55 | CH=CH | 2-$OCH_3$-$C6H_4$ |
| 56 | CH=CH | 3-$OCH_3$-$C_6H_4$ |
| 57 | CH=CH | 4-$OCH_3$-$C_6H_4$ |
| 58 | CH=CH | 2-$CF_3$-$C_6H_4$ |
| 59 | CH=CH | 3-$CF_3$-$C_6H_4$ |
| 60 | CH=CH | 4-$CF_3$-$C_6H_4$ |
| 61 | CH=CH | 4-i-$C_3H_7$-$C_6H_4$ |
| 62 | CH=CH | 4-t-$C_4H_9$-$C_6H_4$ |
| 63 | CH=CH | 4-$C_6H_5$-$C_6H_4$ |
| 64 | CH=CH | 2,4-$Cl_2$-$C_6H_3$ |
| 65 | CH=CH | 2,4-$(CH_3)_4$-$C_6H_3$ |
| 66 | CH=CH | 2,4,6-$(CH_3)_3$-$C_6H_2$ |
| 67 | CH=CH | 2,4,6-$Cl_3$-$C_6H_2$ |
| 68 | CH=CH | Pyridin-2-yl |
| 69 | CH=CH | Pyridin-3-yl |

| Nr. | Y | $R^1$ |
|---|---|---|
| 70 | CH=CH | Furan-2-yl |
| 71 | CH=CH | 6-$CH_3$-Pyridin-2-yl |
| 72 | CH=CH | 6-Cl-Pyridin-2-yl |
| 73 | CH=CH | Benzothiazol-2-yl |
| 74 | $CH_2O$ | $C_6H_5$ |
| 75 | $CH_2O$ | 2-F-$C_6H_4$ |
| 76 | $CH_2O$ | 3-F-$C_6H_4$ |
| 77 | $CH_2O$ | 4-F-$C_6H_4$ |
| 78 | $CH_2O$ | 2-Cl-$C_6H_4$ |
| 79 | $CH_2O$ | 3-Cl-$C_6H_4$ |
| 80 | $CH_2O$ | 4-Cl-$C_6H_4$ |
| 81 | $CH_2O$ | 2-Br-$C_6H_4$ |
| 82 | $CH_2O$ | 3-Br-$C_6H_4$ |
| 83 | $CH_2O$ | 4-Br-$C_6H_4$ |
| 84 | $CH_2O$ | 2-3-$C_6H_4$ |
| 85 | $CH_2O$ | 2-$CH_3$-$C_6H_4$ |
| 86 | $CH_2O$ | 3-$CH_3$-$C_6H_4$ |
| 87 | $CH_2O$ | 4-$CH_3$-$C_6H_4$ |
| 88 | $CH_2O$ | 2-$OCH_3$-$C_6H_4$ |
| 89 | $CH_2O$ | 3-$OCH_3$-$C_6H_4$ |
| 90 | $CH_2O$ | 4-$OCH_3$-$C_6H_4$ |
| 91 | CH2O | 2-$CF_3$-$C_6H_4$ |
| 92 | $CH_2O$ | 3-$CF_3$-$C_6H_4$ |
| 93 | $CH_2O$ | 4-$CF_3$-$C_6H_4$ |
| 94 | $CH_2O$ | 4-i-$C_3H_7$-$C_6H_4$ |
| 95 | $CH_2O$ | 4-t-$C_4H_9$-$C_6H_4$ |
| 96 | $CH_2O$ | 4-$C_6H_5$-$C_6H_4$ |
| 97 | $CH_2O$ | 2,4-$Cl_2$-$C_6H_3$ |
| 98 | $CH_2O$ | 2,4-$(CH_3)_4$-$C_6H_3$ |
| 99 | $CH_2O$ | 2,4,6-$(CH_3)_3$-$C_6H_2$ |
| 100 | $CH_2O$ | 2,4,6-$Cl_3$-$C_6H_2$ |
| 101 | $CH_2O$ | Pyridin-2-yl |
| 102 | $CH_2O$ | Pyridin-3-yl |
| 103 | $CH_2O$ | Furan-2-yl |
| 104 | $CH_2O$ | 6-$CH_3$-Pyridin-2-yl |
| 105 | $CH_2O$ | 6-Cl-Pyridin-2-yl |
| 106 | $CH_2O$ | Benzothiazol-2-yl |
| 107 | $OCH_2$ | H |
| 108 | $OCH_2$ | $C_6H_5$ |

| Nr. | Y | $R^1$ |
|---|---|---|
| 109 | $OCH_2$ | $2\text{-F-}C_6H_4$ |
| 110 | $OCH_2$ | $3\text{-F-}C_6H_4$ |
| 111 | $OCH_2$ | $4\text{-F-}C_6H_4$ |
| 112 | $OCH_2$ | $2\text{-Cl-}C_6H_4$ |
| 113 | $OCH_2$ | $3\text{-Cl-}C_6H_4$ |
| 114 | $OCH_2$ | $4\text{-Cl-}C_6H_4$ |
| 115 | $OCH_2$ | $2\text{-Br-}C_6H_4$ |
| 116 | $OCH_2$ | $3\text{-Br-}C_6H_4$ |
| 117 | $OCH_2$ | $4\text{-Br-}C_6H_4$ |
| 118 | $OCH_2$ | $2\text{-J-}C_6H_4$ |
| 119 | $OCH_2$ | $2\text{-}CH_3\text{-}C_6H_4$ |
| 120 | $OCH_2$ | $3\text{-}CH_3\text{-}C_6H_4$ |
| 121 | $OCH_2$ | $4\text{-}CH_3\text{-}C_6H_4$ |
| 122 | $OCH_2$ | $2\text{-}OCH_3\text{-}C_6H_4$ |
| 123 | $OCH_2$ | $3\text{-}OCH_3\text{-}C_6H_4$ |
| 124 | $OCH_2$ | $4\text{-}OCH_3\text{-}C_6H_4$ |
| 125 | $OCH_2$ | $2\text{-}CF_3\text{-}C_6H_4$ |
| 126 | $OCH_2$ | $3\text{-}CF_3\text{-}C_6H_4$ |
| 127 | $OCH_2$ | $4\text{-}CF_3\text{-}C_6H_4$ |
| 128 | $OCH_2$ | $2\text{-}NO_2\text{-}C_6H_4$ |
| 129 | $OCH_2$ | $4\text{-}NO_2\text{-}C_6H_4$ |
| 130 | $OCH_2$ | $2\text{-}CH_2Cl\text{-}C_6H_4$ |
| 131 | $OCH_2$ | $3\text{-}CH_2Cl\text{-}C_6H_4$ |
| 132 | $OCH_2$ | $4\text{-}CH_2Cl\text{-}C_6H_4$ |
| 133 | $OCH_2$ | $2\text{-}C_2H_5\text{-}C_6H_4$ |
| 134 | $OCH_2$ | $3\text{-}C_2H_5\text{-}C_6H_4$ |
| 135 | $OCH_2$ | $4\text{-}C_2H_5\text{-}C_6H_4$ |
| 136 | $OCH_2$ | $3\text{-i-}C_3H_7\text{-}C_6H_4$ |
| 137 | $OCH_2$ | $4\text{-i-}C_3H_7\text{-}C_6H_4$ |
| 138 | $OCH_2$ | $3\text{-t-}C_4H_9\text{-}C_6H_4$ |
| 139 | $OCH_2$ | $4\text{-t-}C_4H_9\text{-}C_6H_4$ |
| 140 | $OCH_2$ | $3\text{-}C_6H_5\text{-}C_6H_4$ |
| 141 | $OCH_2$ | $4\text{-}C_6H_5\text{-}C_6H_4$ |
| 142 | $OCH_2$ | $4\text{-t-}C_3H_7O\text{-}C_6H_4$ |
| 143 | $OCH_2$ | $4\text{-t-}C_4H_9O\text{-}C_6H_4$ |
| 144 | $OCH_2$ | $3\text{-}C_6H_5O\text{-}C_6H_4$ |
| 145 | $OCH_2$ | $4\text{-}C_6H_5O\text{-}C_6H_4$ |
| 146 | $OCH_2$ | $3\text{-}C_6H_5CH_2O\text{-}C_6H_4$ |
| 147 | $OCH_2$ | $4\text{-}C_6H_5CH_2O\text{-}C_6H_4$ |

| Nr. | Y | $R^1$ |
|---|---|---|
| 148 | $OCH_2$ | $2,3-Cl_2-C_6H_3$ |
| 149 | $OCH_2$ | $2,4-Cl_2-C_6H_3$ |
| 150 | $OCH_2$ | $2,5-Cl_2-C_6H_3$ |
| 151 | $OCH_2$ | $2,6-Cl_2-C_6H_3$ |
| 152 | $OCH_2$ | $2,3,4-Cl_3-C_6H_2$ |
| 153 | $OCH_2$ | $2,3,5-Cl_3-C_6H_2$ |
| 154 | $OCH_2$ | $2,3,6-Cl_3-C_6H_2$ |
| 155 | $OCH_2$ | $C_6Cl_5$ |
| 156 | $OCH_2$ | $C_6F_5$ |
| 157 | $OCH_2$ | 2-F, $4-Cl-C_6H_3$ |
| 158 | $OCH_2$ | 4-F, $2-Cl-C_6H_3$ |
| 159 | $OCH_2$ | $2-CH_3$, $4-t-C_4H_9-C_6H_3$ |
| 160 | $OCH_2$ | $2-CH_3$, 4-Cyclohexyl-$C_6H_3$ |
| 161 | $OCH_2$ | $2-CH_2$, $4-i-C_3H_7$,$-C_6H_3$ |
| 162 | $OCH_2$ | $2,3-(CH_3)_2-C_6H_3$ |
| 163 | $OCH_2$ | $2,4-(CH_3)_2-C_6H_3$ |
| 164 | $OCH_2$ | $2,5-(CH_3)_2-C_6H_3$ |
| 165 | $OCH_2$ | $2,3,5-(CH_3)_3-C_6H_2$ |
| 166 | $OCH_2$ | $2,4,6-(CH_3)_3-C_6H_2$ |
| 167 | $OCH_2$ | $3,4-(CH_3)_2-C_6H_3$ |
| 168 | $OCH_2$ | $3,5-(CH_3)_2-C_6H_3$ |
| 169 | $OCH_2$ | $3,5-(C_2H_5)_2-$ |
| 170 | $OCH_2$ | 4-Cyclohexyl-$C_6H_4$ |
| 171 | $OCH_2$ | $CH_2CH=CH_2$ |
| 172 | $OCH_2$ | $CH_2-CH=CHCH_3$ |
| 173 | $OCH_2$ | $CH_2=CH=C(CH_3)_2$ |
| 174 | $OCH_2$ | $CH_2-C(CH_3)=CH_2$ |
| 175 | $OCH_2$ | $CH_2-C_6H_5$ |
| 176 | $OCH_2$ | Cyclohexyl |
| 177 | $OCH_2$ | $CH_2-C=CH$ |
| 178 | $OCH_2$ | $CH_2CH=CH-C_6H_5$ |
| 179 | $OCH_2$ | $CH_2CH_2-O-C_6H_5$ |
| 180 | O | H |
| 181 | O | $C_6H_5$ |
| 182 | O | $3-C_6H_5-C_6H_4$ |
| 183 | O | $3-n-C_3H_7O-C_6H_4$ |
| 184 | O | Pyridin-2-yl |
| 185 | O | $6-C_6H_5$-Pyridin-2-yl |
| 186 | O | $CH_2-CH=CH_2$ |

| Nr. | Y | $R^1$ |
|---|---|---|
| 187 | O | $3-C_6H_5O-C_6H_4$ |
| 188 | O | $3-C_6H_5-s-C_6H_4$ |
| 189 | O | $3-C_6H_5-CH_2O-C_6H_4$ |
| 190 | O | $4-C_6H_5O-C_6H_4$ |
| 191 | O | $4-C_6H_5OCH_2-C_6H_4$ |
| 192 | C=C | $CH_3$ |
| 193 | C=C | $C_6H_5$ |
| 194 | S | $C_6H_5$ |
| 195 | S | $2-Cl-C_6H_4$ |
| 196 | $SCH_2$ | $C_6H_5$ |
| 197 | $SCH_2$ | $2-Cl-C_6H_4$ |
| 198 | $SCH_2$ | $4-Cl-C_6H_4$ |
| 199 | $SCH_2$ | $4-F-C_6H_4$ |
| 200 | $SCH_2$ | $4-CH_3-C_6H4$ |
| 201 | $SCH_2$ | $6-CH_3$-Pyridin-2-yl |
| 202 | SCH2 | 6-Cl-Pyridin-2-yl |
| 203 | $SCH_2$ | Benzothiazol-2-yl |
| 204 | $SCH_2$ | 5-Cl-Benzothiazol-2-yl |
| 205 | $SCH_2$ | 6-Cl-Benzothiazol-2-yl |
| 206 | $SCH_2$ | $4,8-(CH_3)_2$-Chinolin-2-yl |
| 207 | CO-O | $CH_3$ |
| 208 | Co-o | $C_6H_5$ |
| 209 | O-CO | $CH_3$ |
| 210 | O-CO | $C_6H_5$ |
| 211 | O-CO | $CH_2C_6H_5$ |
| 212 | O-CO | H |
| 213 | $CO-CH_2$ | H |
| 214 | $CO-CH_2$ | $CH_3$ |
| 215 | $CO-CH_2$ | $C_6H_5$ |
| 216 | $CO-CH_2$ | $2-CH_3-C_6H_4$ |
| 217 | $CO-CH_2$ | $2,4-(CH_3)_2-C_6H_3$ |
| 218 | $CO-CH_2$ | $2-Cl-C_6H_4$ |
| 219 | $CH_2-CO$ | H |
| 220 | $CH_2-CO$ | $C_6H_5$ |
| 221 | N=N | $C_6H_5$ |

**Patentansprüche**

1. Ortho-substituierte Phenylessigsäureamide der allgemeinen Formel I,

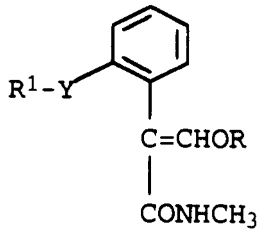

I,

wobei die Variablen folgende Bedeutung haben:

R eine $C_1$-$C_4$-Alkylgruppe,

$R^1$ Wasserstoff, eine $C_1$-$C_{18}$-Alkylgruppe,
eine $C_3$-$C_8$-Cycloalkylgruppe die noch einen bis drei Substituenten tragen kann, ausgewählt aus einer Gruppe von 3 Halogenatomen, 3 $C_1$-$C_4$-Alkylgruppen, einer partiell oder vollständig halogenierten $C_2$-$C_4$-Alkenylgruppe und einer Phenylgruppe, die noch ein bis zwei Halogenatome und/oder eine $C_1$-$C_4$-Alkylgruppe und/oder eine $C_1$-$C_4$-Alkoxygruppe tragen kann,
eine $C_2$-$C_{10}$-Alkenylgruppe,
eine $C_2$-$C_4$-Alkinylgruppe, die einen Phenylrest tragen kann,
eine $C_1$-$C_4$-Alkoxy-$C_1$-$C_4$-alkylgruppe, eine $C_1$-$C_4$-Alkoxycarbonylgruppe,
die Phenylgruppe, eine Phenyl-$C_1$-$C_4$-alkyl- oder Phenyl-$C_2$-$C_4$-alkenylgruppe oder eine Phenoxy-$C_1$-$C_4$-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: $C_1$-$C_4$-Alkyl, partiell oder vollständig halogeniertes $C_1$-$C_4$-Alkyl, $C_2$-$C_4$-Alkenyl, partiell oder vollständig halogeniertes $C_2$-$C_4$-Alkenyl und $C_1$-$C_4$-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder $C_1$-$C_4$-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anneliert sein kann und wobei der Heterocyclus zusätzlich noch ein Halogenatom, einen oder zwei $C_1$-$C_4$-Alkylreste oder einen Phenylrest tragen kann;

Y Sauerstoff, Schwefel,
eine Gruppe -SO-, $-SO_2-$, $-CH_2-O-SO_2-$, -N = N-, -O-CO- oder -CO-O-,
eine $C_1$-$C_4$-Alkylenkette, die partiell oder vollständig halogeniert sein kann, und die noch zusätzlich einen der folgenden Reste tragen kann: Cyano, Nitro, $C_1$-$C_4$-Alkyl,partiell oder vollständig halogeniertes $C_1$-$C_4$-Alkyl, $C_2$-$C_4$-Alkenyl, partiell oder vollständig halogeniertes $C_2$-$C_4$-Alkenyl, $C_1$-$C_4$-Alkoxy, Phenyl oder Phenoxy, wobei die letzten beiden Reste ihrerseits noch einen oder zwei der folgenden Substituenten tragen können: Cyano, Halogen oder $C_1$-$C_4$-Alkyl,
eine $C_2$-$C_4$-Alkenylen- oder $C_2$-$C_4$-Alkinylenkette, eine Oxy-$C_1$-$C_4$-alkylen-, Thio-$C_1$-$C_4$-alkylen- oder $C_1$-$C_4$-Alkylenoxykette oder eine Carbonyl-$C_1$-$C_4$-alkylen- oder $C_1$-$C_4$-Alkylencarbonylkette.

2. Verfahren zur Herstellung der Verbindungen I gemäß Anspruch 1, dadurch gekennzeichnet, daß man ein Phenylessigsäurederivat der Formel II

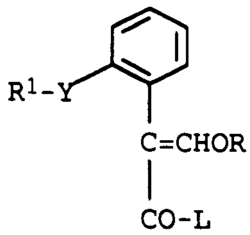

II,

wobei L Halogen oder $C_1$-$C_4$-Alkoxy bedeutet, gewünschtenfalls in Gegenwart einer Base mit Methylamin ($H_2NCH_3$, III) umsetzt.

**3.** Verfahren zur Herstellung der Verbindungen I gemäß Anspruch 1, dadurch gekennzeichnet, daß man ein Phenylacetonitril der Formel IV

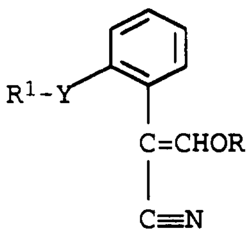

IV,

in Gegenwart einer Säure oder Base hydrolysiert und das Verfahrensprodukt am Amidstickstoff einmal methyliert.

**4.** Fungizides Mittel, enthaltend einen flüssigen oder festen Trägerstoff und mindestens eine Verbindung der Formel I gemäß Anspruch 1.

**5.** Schädlingsbekämpfungsmittel, enthaltend einen inerten Trägerstoff und mindestens eine Verbindung der Formel I gemäß Anspruch 1.

**6.** Verfahren zur Bekämpfung von Pilzen, dadurch gekennzeichnet, daß man eine fungizid wirksame Menge einer Verbindung der Formel I gemäß Anspruch 1 auf Pilze, von Pilzbefall bedrohte Pflanzen, deren Lebensraum oder auf das Saatgut der bedrohten Pflanzen einwirken läßt.

**7.** Verfahren zur Bekämpfung von Schädlingen, dadurch gekennzeichnet, daß man eine insektizid, nematizid und/oder akarizid wirksame Menge einer Verbindung der Formel I gemäß Anspruch 1 auf Insekten, Nematoden und/oder Akaridien bzw. deren Lebensraum einwirken läßt.

**8.** Verbindungen der Formel I gemäß Anspruch 1, wobei die Variablen die folgende Bedeutung haben:

R Methyl,

$R^1$ Phenyl, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: $C_1$-$C_4$-Alkyl, partiell oder vollständig halogeniertes $C_1$-$C_4$-Alkyl, $C_2$-$C_4$-Alkenyl, partiell oder vollständig halogeniertes $C_2$-$C_4$-Alkenyl und $C_1$-$C_4$-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder $C_1$-$C_4$-Alkyl; und

Y $-OCH_2-$.

**9.** Verbindungen der Formel I gemäß Anspruch 1, in denen Y eine der folgenden Gruppen bedeutet: -O-, -S-, -O-CO-, -CO-O-, $-CH_2-$, $-CH_2CH_2-$, -CH=CH-, -C≡C-, $-OCH_2-$, $-SCH_2-$, $-CH_2O-$, $-CO-CH_2-$ oder $-CH_2-CO-$.

## Claims

**1.** An ortho-substituted phenylacetamide of the formula I

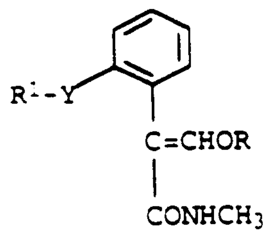

**(I)**

where

R is $C_1$-$C_4$-alkyl,

$R^1$ is hydrogen, $C_1$-$C_{18}$-alkyl,

$C_3$-$C_8$-cycloalkyl which can have from one to three substituents selected from a group of 3 halogen atoms, 3 $C_1$-$C_4$-alkyl groups, a partially or completely halogenated $C_2$-$C_4$-alkenyl group and a phenyl group which can carry one or two halogen atoms and/or one $C_1$-$C_4$-alkyl group and/or one $C_1$-$C_4$-alkoxy group,

$C_2$-$C_{10}$-alkenyl,

$C_2$-$C_4$-alkynyl which can carry a phenyl radical,

$C_1$-$C_4$-alkoxy-$C_1$-$C_4$-alkyl, $C_1$-$C_4$-alkoxycarbonyl,

phenyl, phenyl-$C_1$-$C_4$-alkyl or phenyl-$C_2$-$C_4$-alkenyl or phenoxy-$C_1$-$C_4$-alkyl where each aromatic ring can have from one to five substituents selected from a group of 2 nitro radicals, 2 cyano radicals, 5 halogen atoms and in each case three of the following: $C_1$-$C_4$-alkyl, partially or completely halogenated $C_1$-$C_4$-alkyl, $C_2$-$C_4$-alkenyl, partially or completely halogenated $C_2$-$C_4$-alkenyl and $C_1$-$C_4$-alkoxy, and of one phenyl, benzyl, phenoxy, benzyloxy or phenylthio radical, where the last two radicals in turn can have one or two of the following substituents: cyano, halogen or $C_1$-$C_4$-alkyl;

a 5- or 6-membered heterocycle with from one to three hetero atoms selected from a group of two oxygen, two sulfur and three nitrogen atoms, excepting compounds with two adjacent oxygen and/or sulfur atoms, it being possible for a benzene ring or a 5- or 6-membered heteroaromatic ring with one nitrogen, oxygen or sulfur atom to be fused onto the heterocycle, and it being possible for the heterocycle to carry one halogen atom, one or two $C_1$-$C_4$-alkyl radicals or one phenyl radical;

Y is oxygen, sulfur,

-SO, $-SO_2-$, $-CH_2-O-SO_2-$, -N = N-, -O-CO- or -CO-O-,

$C_1$-$C_4$-alkylene which can be partially or completely halogenated and can additionally carry one of the following: cyano, nitro, $C_1$-$C_4$-alkyl, partially or completely halogenated $C_1$-$C_4$-alkyl, $C_2$-$C_4$-alkenyl, partially or completely halogenated $C_2$-$C_4$-alkenyl, $C_1$-$C_4$-alkoxy, phenyl or phenoxy, it being possible for the last two radicals in turn to have one or two of the following substituents: cyano, halogen or $C_1$-$C_4$-alkyl,

$C_2$-$C_4$-alkenylene or $C_2$-$C_4$-alkynylene, oxy-$C_1$-$C_4$-alkylene, thio-$C_1$-$C_4$-alkylene or $C_1$-$C_4$-alkyleneoxy or carbonyl-$C_1$-$C_4$-alkylene or $C_1$-$C_4$-alkylenecarbonyl.

**2.** A process for preparing a compound I as claimed in claim 1, which comprises reacting a phenylacetic acid derivative of the formula II

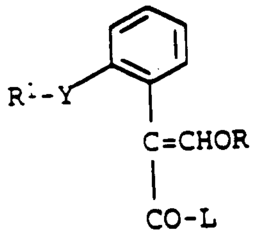

(II)

where L is halogen or $C_1$-$C_4$-alkoxy, if desired in the presence of a base, with methylamine ($H_2NCH_3$, III).

**3.** A process for preparing a compound I as claimed in claim 1, which comprises hydrolyzing a phenylacetonitrile of the formula IV

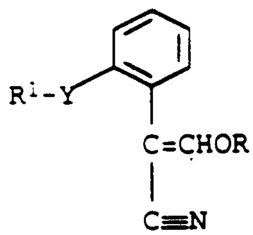

(IV)

in the presence of an acid or base, and methylating the product once on the amide nitrogen.

**4.** A fungicidal agent containing a liquid or solid carrier and at least one compound of the formula I as claimed in claim 1.

**5.** A pesticide containing an inert carrier and at least one compound of the formula I as claimed in claim 1.

**6.** A method for controlling fungi, which comprises exposing the fungi, the plants threatened by fungal attack, their habitat or the seed of the threatened plants to a fungicidal amount of a compound of the formula I as claimed in claim 1.

**7.** A method for controlling pests, which comprises exposing insects, nematodes and/or acarids or their habitat to an insecticidal, nematicidal and/or acaricidal amount of a compound of the formula I as claimed in claim 1.

**8.** A compound of the formula I as claimed in claim 1, where

- R is methyl,
- $R^1$ is phenyl where each aromatic ring can have from one to five substituents selected from a group of 2 nitro radicals, 2 cyano radicals, 5 halogen atoms and in each case three of the following: $C_1$-$C_4$-alkyl, partially or completely halogenated $C_1$-$C_4$-alkyl, $C_2$-$C_4$-alkenyl, partially or completely halogenated $C_2$-$C_4$-alkenyl and $C_1$-$C_4$-alkoxy, and of one phenyl, benzyl, phenoxy, benzyloxy or phenylthio radical, where the last two radicals in turn can have one or two of the following substituents: cyano, halogen or $C_1$-$C_4$-alkyl; and
- Y is $-OCH_2-$.

**9.** A compound of the formula I as claimed in claim 1, where Y is one of the following: -O-, -S-, -O-CO-, -CO-O-, $-CH_2$, $-CH_2CH_2-$, -CH = CH, -C≡C-, $-OCH_2-$, $-SCH_2-$, $-CH_2O-$, $-CO-CH_2-$ or $-CH_2-CO-$.

## Revendications

1. Amides d'acide phénylacétique substitués en ortho, de formule générale I

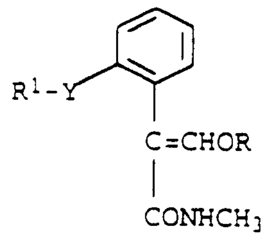

I,

où les variables ont les significations suivantes :

R un groupe alkyle en C1-C4,

$R^1$ hydrogène, un groupe alkyle en C1-C18,

un groupe cycloalkyle en C3-C8 qui peut encore porter un à trois substituants, choisis dans le groupe constitué de 3 atomes d'halogène, 3 groupes alkyle en C1-C4, un groupe alcényle en C2-C4, partiellement ou complètement halogéné, et un groupe phényle pouvant encore porter un ou deux atomes d'halogène et/ou un groupe alkyle en C1-C4 et/ou un groupe alcoxy en C1-C4,

un groupe alcényle en C2-C10,

un groupe alcynyle en C2-C4 pouvant porter un reste phényle,

un groupe alcoxy en C1-C4-alkyle en C1-C4, un groupe alcoxy en C1-C4-carbonyle,

le groupe phényle, un groupe phényl-alkyle en C1-C4 ou phényl-alcényle en C2-C4 ou un groupe phénoxy-alkyle en C1-C4, l'aromatique pouvant dans chaque cas encore porter un à cinq substituants choisis dans le groupe constitué de 2 restes nitro, 2 restes cyano, 5 atomes d'halogène et dans chaque cas trois des restes suivants : alkyle en C1-C4, alkyle en C1-C4 partiellement ou complètement halogéné, alcényle en C2-C4, alcényle en C2-C4 partiellement ou complètement halogéné, et alcoxy en C1-C4 et un reste phényle, benzyle, phénoxy, benzyloxy ou phénylthio, les deux derniers restes cités pouvant eux-mêmes encore porter un ou deux des substituants suivants : cyano, halogène ou alkyle en C1-C4 ;

un hétérocycle à 5 ou 6 chaînons portant un à trois hétéroatomes, choisis dans le groupe constitué de deux atomes d'oxygène, deux atomes de soufre et trois atomes d'azote, les composés à deux atomes d'oxygène et/ou de soufre contigus étant exceptés, un noyau benzénique ou un hétéroaromatique à 5 ou 6 chaînons portant un atome d'azote, d'oxygène ou de soufre pouvant encore être accolés à l'hétérocycle et l'hétérocycle pouvant porter additionnellement un ou deux restes alkyle en C1-C4 ou un reste phényle ;

Y oxygène, soufre,

un groupe -SO-, $-SO_2-$, $-CH-O-SO_2$, -N = N-, -O-CO- ou -CO-O-,

une chaîne alkylène en C1-C4 pouvant être halogénée partiellement ou complètement et pouvant porter additionnellement un des restes suivants : cyano, nitro, alkyle en C1-C4, alkyle en C1-C4 partiellement ou complètement halogéné, alcényle en C2-C4, alcényle en C2-C4 partiellement ou complètement halogéné, alcoxy en C1-C4, phényle ou phénoxy, les deux derniers restes cités pouvant encore porter eux-mêmes un ou deux des substituants suivants : cyano, halogène ou alkyle en C1-C4,

une chaîne alcénylène en C2-C4 ou une chaîne alcynylène en C2-C4, une chaîne oxy-alkylène en C1-C4, thioalkylène en C1-C4 ou alkylène en C1-C4-oxy ou une chaîne carbonyl-alkylène en C1-C4 ou alkylène en C1-C4-carbonyle.

2. Procédé de préparation des composés I, selon la revendication 1, caractérisé par le fait que l'on fait réagir, si souhaité en présence d'une base, un dérivé d'acide phénylacétique de formule II

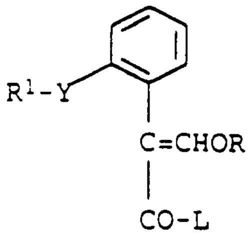

II,

où L représente halogène ou alcoxy en C1-C4, avec de la méthylamine ($H_2NCH_3$, III).

**3.** Procédé de préparation des composés I, selon la revendication 1, caractérisé par le fait que l'on hydrolyse un phénylacétonitrile de formule IV

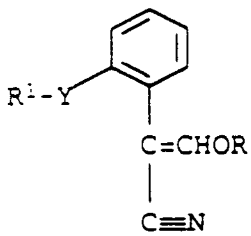

IV,

en présence d'un acide ou d'une base, le produit du procédé étant ensuite méthylé une fois sur l'azote d'amide.

**4.** Fongicide, contenant un véhicule liquide ou solide et au moins un composé de formule I, selon la revendication 1.

**5.** Pesticide contenant un véhicule inerte et au moins un composé de formule I, selon la revendication 1.

**6.** Procédé de lutte contre les champignons, caractérisé par le fait que l'on fait agir une quantité efficace en tant que fongicide d'un composé de formule I, selon la revendication 1, sur les champignons, les plantes menacées par l'attaque par les champignons, le biotope de ces derniers ou les semences des plantes menacées.

**7.** Procédé de lutte contre les parasites, caractérisé par le fait que l'on fait agir une quantité efficace en tant qu'insecticide, nématicide et/ou acaricide d'un composé de formule I, selon la revendication 1, sur les insectes, nématodes et/ou acariens ou leur biotope.

**8.** Composés de formule I, selon la revendication 1, où les variables ont les significations suivantes :
R méthyle,
$R^1$ phényle, l'aromatique pouvant dans chaque cas encore porter un à cinq substituants, choisis dans le groupe constitué de 2 restes nitro, 2 restes cyano, 5 atomes d'halogène et dans chaque cas trois des restes suivants : alkyle en C1-C4, alkyle en C1-C4 partiellement ou complètement halogéné, alcényle en C2-C4, alcényle en C2-C4 partiellement ou complètement halogéné et alcoxy en C1-C4, ainsi qu'un reste phényle, benzyle, phénoxy, benzyloxy ou phénylthio, les deux derniers restes cités pouvant porter eux-mêmes encore un ou deux des substituants suivants : cyano, halogène ou alkyle en C1-C4 ; et
Y $-OCH_2-$.

**9.** Composés de formule I, selon la revendication 1, dans lesquels Y représente un des groupes suivants : -O-, -S-, -O-CO-, -CO-O-, $-CH_2-$, $-CH_2CH_2-$, -CH=CH-, -C≡C-, $-OCH_2-$, $-SCH_2-$, $-CH_2O-$, $-CO-CH_2-$ ou $-CH_2-CO-$.